# EUROPEAN PATENT APPLICATION

(11) **EP 1 080 722 A1**
(43) Date of publication of application: **07.03.2001**
(21) Application number: 99117111.7
(22) Date of filing: 31.08.1999
(51) Int. Cl.: A61K 31/136, A61P 31/10

(54) **Antimycotic compositions**

(71) Applicant: Novartis AG, 4058 Basel (CH)
(72) Inventor: The designation of the inventor has not yet been filed
(74) Representative: Becker, Konrad

(57) **Abstract**

Described is the excellent and safe antimycotic action of the compound of the formula (1) 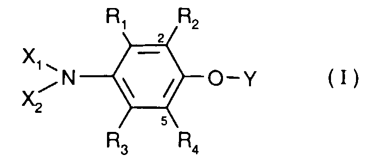 wherein Y is a completely or partially halogenated C₁-C₆alkyl or a completely or partially halogenated and with an oxygen atom interrupted C₁-C₆alkyl or a completely or partially halogenated C₂-C₆alkenyl; R₁, R₂, R₃ and R₄ are independently of each other hydrogen, halogen, C₁-C₆alkyl, C₁-C₆haloalkyl, C₁-C₆haloalkoxy, X₁ is hydrogen or C₁-C₆alkyl, X₂ is hydrogen or C₁-C₆alkyl. Further described are antimycotic compositions comprising as active ingredient at least one of said compounds, and to the use of said compounds and antimycotic compositions for the treatment of mycotic infections in humans or animals.

## Description

The present invention relates to the excellent and safe antimycotic action of the compound of the formula ( I ) that are defined below. It further relates to antimycotic compositions comprising as active ingredient at least one of said compounds, and to the use of said compounds and antimycotic compositions for the treatment of mycotic infections in humans or animals. A further aspect of the present invention is the preparation of said antimycotic compositions as well as the use of said compounds and compositions in the prevention or treatment of mycotic infections in humans or animals.

It has surprisingly been found that the compounds of the formula ( I ) 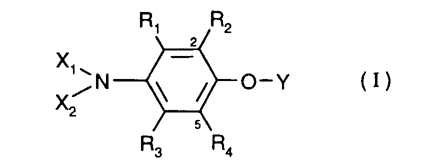 wherein Y is a completely or partially halogenated C₁-C₆alkyl or a completely or partially halogenated and with an oxygen atom interrupted C₁-C₆alkyl or a completely or partially halogenated C₂-C₆alkenyl; R₁, R₂, R₃ and R₄ are independently of each other hydrogen, halogen, C₁-C₆alkyl, C₁-C₆haloalkyl, C₁-C₆haloalkoxy, X₁ is hydrogen or C₁-C₆alkyl, X₂ is hydrogen or C₁-C₆alkyl, not only exhibit excellent and safe antimycotic properties against a broad range of fungi and yeasts but can also be administered systemically or topically, and can be combined with other antimycotics to treat resistant pathogens.

Typical and preferred representatives of the substituent Y are: CF₃, C₂F₅, C₃F₇, C₄F₉, C₅F₁₁, C₆F₁₃, CF(CF₃)₂, CF(C₂F₅)(CF₃), CF(C₂F₅)(C₂F₅), CF₂OCF₃, CF₂OCF(C₂F₅)₂, CF₂CHFCF₃, CH(CF₃)CF₂CF₃, CH(CF₃)CF₂C₂F₅, CH(CH₃)CF₂CF₃, CH(CH₃)CF₂C₂F₅, CF=CFCF₃, CF₂CF=CFCF₃, CF₂(CF₃)CF₂=CFCF₃,OCF₂(CF₃)-O-CF₂=CFCF₃, CF₂CFHOCF₃, CF₂CCl₃, CF₂CHCl₂, CF₂CHF₂, CF₂CFCl₂, CF₂CHBr₂, CF₂CHClF, CH₂CHBrCH₂Br, CF₂CHBrF, and CClFCHClF. More preferred are CF₂CHFCF₃, CH(CF₃)CF₂CF₃, CH(CF₃)CF₂C₂F₅, CH(CH₃)CF₂CF₃, CH(CH₃)CF₂C₂F₅, CF=CFCF₃, CF₂CF=CFCF₃, CF₂CHF₂, and CF₂CFHOCF₃. Most preferred is CF₂CHFCF₃. Typical and preferred representatives of the substituents R₁, R₂, R₃, and R₄ are H, F, Cl, Br, CH₃, C₂H₅, and CF₃. Most preferred is for R₁ H, F, Cl, Br, CH₃ or CF₃; for R₂ H, Cl, Br or CF₃, for R₃ H, F, Cl, Br or CH₃, and for R₄ H, Cl or Br. Typical and preferred representatives of the substituents X₁ and X₂ are H, CH₃ and C₂H₅; most preferred is H.

Due to their antimycotic properties the compounds of the following groups 1 to 4 represent preferred embodiments of the present invention:
1) Compounds of the formula ( I ) wherein Y is CF₃, C₂F₅, C₃F₇, C₄F₉, C₅F₁₁, C₆F₁₃, CF(CF₃)₂, CF(C₂F₅)(CF₃), CF(C₂F₅)(C₂F₅), CF₂OCF₃, CF₂OCF(C₂F₅)₂, CF₂CHFCF₃, CH(CF₃)CF₂CF₃, CH(CF₃)CF₂C₂F₅, CH(CH₃)CF₂CF₃, CH(CH₃)CF₂C₂F₅, CF=CFCF₃, CF₂CF=CFCF₃, CF₂(CF₃)CF₂=CFCF₃, OCF₂(CF₃)-O-CF₂=CFCF₃, CF₂CFHOCF₃, CF₂CCl₃, CF₂CHCl₂, CF₂CHF₂, CF₂CFCl₂, CF₂CHBr₂, CF₂CHCIF, CH₂CHBrCH₂Br, CF₂CHBrF or CClFCHClF, X₁ and X₂ are independently of each other H, CH₃ or C₂H₅; and R₁, R₂, R₃ and R₄ are as defined as under formula ( I ). Most preferred within this group are those compounds wherein R₁, R₂, R₃, and R₄ are independently of each other H, F, Cl, Br, CH₃, C₂H₅, or CF₃.
2) Compounds of the formula ( I ) wherein Y is CF₂CHFCF₃, CH(CF₃)CF₂CF₃, CH(CF₃)CF₂C₂F₅, CH(CH₃)CF₂CF₃, CH(CH₃)CF₂C₂F₅, CF=CFCF₃, CF₂CF=CFCF₃, CF₂CHF₂, or CF₂CFHOCF₃; X₁ and X₂ are independently of each other H, CH₃ or C₂H₅; and R₁, R₂, R₃ and R₄ are H, F, Cl, Br, CH₃, C₂H₅, or CF₃.
3) Compounds of the formula ( I ) wherein Y is CF₂CHFCF₃, CH(CF₃)CF₂CF₃, CH(CF₃)CF₂C₂F₅, CH(CH₃)CF₂CF₃, CH(CH₃)CF₂C₂F₅, CF=CFCF₃, CF₂CF=CFCF₃, CF₂CHF₂, or CF₂CFHOCF₃; X₁ and X₂ are independently of each other H, CH₃ and C₂H₅; R₁ is H, F, Cl, Br, CH₃ or CF₃; R₂ is H, Cl, Br or CF₃, R₃ is H, F, Cl, Br or CH₃, and R₄ is H, Cl or Br. Most preferred within this group are those compounds wherein X₁ and X₂ are H.
4) Preferred single representatives are: 3-chloro-4-(1,2,2-trifluoro-2-trifluoromethoxyethoxy)aniline; 2-fluoro-3,5-dichloro-4-(1-methyl-2,2,3,3,3-pentafluoropropoxy)aniline; 3,5-dichloro-4-(1,1,2,3,3,3-hexafluoropropoxy)aniline; 3,5-dichloro-4-(1,1,2,3,3,3-hexafluoropropoxy)-N-ethyl-aniline; 3,5-dichloro-4-trans-(1,1,2,3,4,4,4-heptafluorobut-2-enoxy)aniline; 3,5-dichloro-4-(1,1,2,3,3,3-hexafluoropropoxy)aniline); and especially 2,5-dichloro-4-(1,1,2,3,3,3-hexafluoropropoxy)aniline.

Mycosis can be classified into two main types. The first type embraces superficial mycosis represented by various dermatophytoses, cutaneous candidiasis and the like. The second type embraces deep seated mycosis represented by mycotic meningitis, mycotic infectious disease of respiratory organ, fungemia, mycosis of urinary tract and the like. Incidence of candidiasis, aspergillosis and other systemic mycoses shows a marked increase in recent years owing to the frequent use of anticancer chemotherapeutic agents or immunosuppressive agents as well as the spread of HIV infection. Although several antimycotic agents (e.g., amphotericin B, clotrimazole, miconazole, fluconazole, itraconazole, ketoconazole, terbinafine and nystatin) are currently available, these agents are not completely effective. In some cases, these agents can also lead to drug resistant organisms and can produce adverse side effects. In addition, some are not appropriate for oral or systemic administration. Consequently, there is a need for a new pharmaceutical efficacious against fungi causing such diseases, and especially for agents that show a broad range activity, and that can be used in combination with known antimycotics in order to overcome resistance or to minimize the risk of resistance development.

A typical but non-limiting example of an animal disease that can be successfully controlled by the compounds and compositions of the present invention is dermatophytosis or tinea, commonly known as ringworm. Ringworm or dermatophytosis is an infection of the hair and hair follicles caused by certain types of zoophilic fungi which tend to be found on particular animal hosts such as *Microsporum canis* on cats, dogs, horses and rodents, *Trichophyton verrucosum* on cattle and sheep or *Trichophyton mentagrophytes* on dogs, cats and rodents. The fungus invades the outermost layers of the skin to produce the flakiness and invades the hair follicle which causes the hairs to break off at the surface of the skin. Infested animals can spread most types of ringworm to humans. Ringworm is the most common zoonotic disease transferred from animals to humans.
Ringworm is characterized by circular areas of hair loss. Small areas of the animal's skin may be affected, or infection of large areas of the animal may occur. Cats and dogs infected with ringworm often experience hair loss on their heads or faces. Hair loss can also occur on other, areas of their bodies. Hair that remains is usually short and coarse. Redness and itchiness may develop. Some animals with ringworm will show no apparent lesions but can transmit the infection to others. Other animals may show an allergic reaction which results in severe itching.

Veterinarians and practitioners in the field of human medicine can use a number of methods to diagnose ringworm. The most reliable test is a fungal culture. This involves culturing hairs or scales from the infected animal and observing the culture for growth of dermatophyte fungie. Other means may include using a fluorescent light (Wood's Lamp) to detect certain fungi or examining hair specimens under a microscope.
Conventional treatment of ringworm is time-consuming and requires patience, discipline and a lot of sanitary provisions. It often takes several months. If the environment is contaminated it can be a long-term problem lasting for years. Ringworm treatment for cats and dogs includes clipping the entire hair coat although recent findings showed that in some cases clipping the entire cat or dog actually helps spread ringworm across the body.
As reported in August 1999 in the Internet by Kimberly Meenen, information specialist of the University of Illinois College of Veterinary Medicine, a vaccine for ringworm is now being marketed. However, tests at the University of Wisconsin show that the vaccine does not offer protection against infection. Although the vaccine may decrease redness or other symptoms of ringworm, other therapy would probably be needed to clear the infection.
Humans suffer from several forms of dermatophytosis of the skin, hair and nails, or may contract ringworm from animals such as dogs, cats, and calves. Skin lesions are usually ring-shaped, reddish, and flat. Children or adults with skin lesions should see their doctor for a diagnosis and treatment. Ringworm is moderately contagious, and people may pass it on to other people.
Thus, the use of the compounds of the formula ( I ) for controlling or preventing ringworm and for preparing drugs against this disease represent preferred embodiments of the present invention.

The activity spectrum of the compounds of the formula ( I ) is not restricted to fungi but embraces yeast species as well. Budding yeasts are true fungi of the phylum *Ascomycetes,* class *Hemiascomycetes.* The true yeasts are classified in one main order
*Saccharomycetales,* which includes at least ten families. The classification of yeasts is a specialized field using cell, ascospore, and colony characteristics for distinguishing genera, and physiological characteristics - particularly the ability to ferment individual sugars - to identify species.

Yeasts are heterotrophic, lack chlorophyll, and are characterized by a wide dispersion of natural habitats. Common on plant leaves and flowers, yeasts are also found on the skin surfaces and in the intestinal tracts of warm-blooded animals, where they may live symbiotically or as parasites. In women, who are pregnant or taking antibiotics, an infection of the vagina and vulva caused by a yeastlike fungus *Candida albicans,* is common.

Consequently, there is still a real need for active ingredients for control of superficial as well as systemic infections in man and animals that are caused by fungi or yeasts.

Accordingly, an important objective of the present invention is to provide compounds which have high safety and exhibit antimycotic activity against a broad range of fungi and yeasts. The activity spectrum of the compounds of the formula ( I ) embraces but is not limited to the human and animal pathogenic fungi and yeasts shown listed and discussed in Table 2. Antimycotic compositions containing the compounds of the formula ( I ) as the active principle can be used in curative treatment and in preventive treatment as well. The compounds of the formula ( I ) and the antimycotic compositions containing such a compound can be used in the treatment of fungal infections not only in humans but have also veterinary applications.

A further preferred object of the present invention is thus a method for the control of pathogenic fungi and yeasts in and on humans, domestic animals, livestock and pets, comprising a composition which contains at least one compound of formula ( I ), or a veterinarily acceptable salt thereof, and is administered to the host animal orally, parenterally or by implant at an effective dose. This means that the compounds may alternatively be administered topically in such conventional forms as ointments, tinctures, creams, etc., or parenterally. Topical application in the veterinary field embraces sprays, dips, pour-on and spot-on applications which are well known to those skilled in the art. The concentrations of the active substance will of course vary depending on the compound employed, the treatment desired and the nature of the form etc. In general, however, satisfactory results are obtained e.g. in topical application forms at concentrations of from 0.05 to 5, in particular 0.1 to 1 wt %. Essential to the invention is the fact that the composition of the invention is administered in such a way that the active ingredients which the composition comprises can be taken up in sufficient quantity.

This is achieved with the composition of the invention using different forms of application, e.g. through the oral administration of the composition comprising the active ingredients. Formulation in this case means e.g. presentation in the form of a powder, a tablet, granules, a capsule, an emulsion, a foam, or in a microencapsulated form, etc., although it is not absolutely necessary for the preparation to be given directly to the patient or the animal - it can also be added to a drink or mixed into a meal or if an animal has to be treated it can be mixed to the animal's feed where expedient. Of course, all compositions to be given by the oral route may contain, along with the usual formulation assistants, further additives designed to encourage their uptake, e.g. appropriate aromas and flavours. Because of its simplicity of application, the oral route of administration is one of the preferred objects of this invention. A further form of administration is the parenteral route, e.g. by subcutaneous or intravenous injection, topical application, long-term implantation (depot), or an injection of microcapsules (so-called microspheres).
Oral administration includes e.g. giving animal feed, for example dog or cat food, in which the active ingredientsare already mixed, e.g. in the form of biscuits, chewable tablets, water-soluble capsules or tablets, in a water-soluble form that can be applied in drops onto the feed or in other forms that are miscible with the animal feed. Implants include all devices which can be inserted into the body of the animal for delivery of the substance.
Percutaneous forms of administration include for example subcutaneous, dermal, intramuscular and even intravenous administration of injectable forms. Apart from the usual injection syringes with needles, needleless systems may also be expedient.
Through the selection of a suitable formulation, it is possible to promote the permeability of the active ingredients through the living tissue of the animal and maintain their availability. This is of importance if, for example, one or more poorly soluble active ingredients are used whose solubility needs to be promoted because the body fluid of the animal is only able to dissolve small quantities of the active ingredients at any one time.
Furthermore, the active ingredients may also be present in a matrix formulation, which physically prevents their decomposition and maintains the availability of the active ingredients. This matrix formulation is injected into the body and remains there as a form of depot from which the active ingredients are continuously released. Such matrix formulations are known to persons skilled in the art. They are generally waxlike, semisolid excipients, such as e.g. vegetable waxes and polyethylene glycols of high molecular weight or copolymers from degradable polyesters.

For the above-mentioned use, the dose administered will of course vary depending on the compound employed, mode of administration and treatment desired. However, in general, satisfactory results are obtained when administered at a daily dosage of from 1 to 100 mg/kg of animal body weight, conveniently given once daily, or in divided doses two to four times daily, or in sustained release form. Preferred dosages are 1 to 50 mg/kg of animal body weight for systemic and 0.1% to 10% for topical administration. For the larger mammals, the corresponding daily dosages are in the range of from 70 to 2000 mg, and dosage forms suitable for oral administration comprise from 17.5 to 1000 mg. The invention therefore also concerns a method of treating diseases or infections caused by mycetes using a compound of formula ( I ). The total dose can vary with the same active ingredient both between and within animal species, since the dose depends among other things on the weight and the constitution of the animal.

The compounds of the present invention may be prepared and used in the free base form or in the form of pharmaceutically acceptable salts (acid addition salts or alcoholates). In general the salt forms exhibit the same order of activity as the free base forms. Acids that may be used in preparing acid addition salt forms include by way of illustration hydrochloric, hydrobromic, sulphuric, nitric, fumaric, and naphthaline-1,5-disulphonic acids. Preferred modes of administration are topical, oral and intravenous.

For the formulation of compositions that are to be administered to humans, domestic animals, livestock, and pets, the adjuvants known from the medical and veterinary practice for oral, parenteral and implant forms can be used. The following is a non-exhaustive list of some examples.
Suitable carriers are in particular fillers, such as sugars, e.g. lactose, saccharose, mannitol or sorbitol, cellulose preparations and/or calcium phosphates, e.g. tricalcium phosphate or calcium hydrogen phosphate, in a broader sense also binders, such as starch pastes using e.g. corn, wheat, rice or potato starch, gelatin, tragacanth, methyl cellulose and/or, if desired, disintegrants, such as the above-mentioned starches, in a broader sense also carboxymethyl starch, cross-linked polyvinylpyrrolidone, agar, alginic acid or a salt thereof, such as sodium alginate. Excipients are especially flow conditioners and lubricants, for example silicic acid, talc, stearic acid or salts thereof, such as magnesium or calcium stearate, and/or polyethylene glycol. Tablet cores may be provided with suitable, where appropriate enteric, coatings, using *inter alia* concentrated sugar solutions which may comprise gum arabic, talc, polyvinylpyrrolidone, polyethylene glycol and/or titanium dioxide, or coating solutions in suitable organic solvents or solvent mixtures, or, for the preparation of enteric coatings, solutions of suitable cellulose preparations, such as acetylcellulose phthalate or hydroxypropylmethylcellulose phthalate. Dyes, flavours or pigments may be added to the tablets or tablet coatings, for example for identification purposes or to indicate different doses of active ingredient.
Further orally administrable pharmaceutical compositions include hard capsules consisting of gelatin, and also soft, sealed capsules consisting of gelatin and a plasticizer, such as glycerol or sorbitol. The hard capsules may contain the active ingredient in the form of granules, for example in admixture with fillers, such as lactose, binders, such as starches, and/or glidants, such as talc or magnesium stearate, and where appropriate stabilizers. In soft capsules, the active ingredients are preferably dissolved or suspended in suitable liquids, such as fatty oils, paraffin oil, or liquid polyethylene glycols, and stabilizers may likewise be added. Amongst other forms, capsules which can be both easily chewed and also swallowed whole are preferred.
The formulations suitable for parenteral administration are especially aqueous solutions of the active ingredients in water-soluble form, e.g. water-soluble salts, in the broader sense also suspensions of the active ingredients, such as appropriate oily injectable suspensions using suitable lipophilic solvents or vehicles, such as oils, e.g. sesame oil, or synthetic fatty acid esters, e.g. ethyl oleate, or triglycerides, or aqueous injectable suspensions containing viscosity-increasing agents, e.g. sodium carboxymethyl cellulose, sorbitol and/or dextran, and where appropriate stabilizers.
The compositions of the invention can be prepared in a known manner, e.g. for example by means of conventional mixing, granulating, coating, dissolving or lyophilizing methods. Pharmaceutical compositions for oral administration can be obtained, for example, by combining the active ingredients with solid carriers, granulating a resulting mixture where appropriate, and processing the mixture or granules, if desired or necessary, to form tablets or tablet cores following the addition of suitable excipients.

The invention therefore relates also to antimycotic compositions, such as emulsifiable concentrates, suspension concentrates, directly sprayable or dilutable solutions, coatable pastes, dilute emulsions, spray powders, soluble powders, dispersible powders, wettable powders, dusts, granulates or encapsulated polymers (chosen in accordance with the intended objectives and prevailing circumstances), comprising at least one active ingredient of the invention.
The active ingredient is used in these compositions in pure form and a solid active ingredient e.g. in a specific particle size, or preferably together with - at least - one of the adjuvants conventionally employed in the art of formulation, such as extenders, e.g. solvents or solid carriers, or surface-active compounds (surfactants). For fungal control in humans, domestic animals, livestock, and pets of course only physiologically acceptable adjuvants are used.

Compositions also form part of the invention.

The compound of formula ( I ) are known or can be prepared in a manner known per se, for example, by hydrogenating the suitably substituted nitrobenzene of formula ( II ) 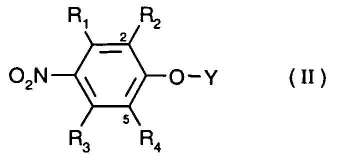 wherein the substituents R₁, R₂, R₃, R₄ and Y are defined as under Formula ( I ) by a process analogous to that described in J. Org. Chem. 29 (1964), 1, (q.v. also the literature cited therein). However, the anilines of formula ( I ) can also be obtained by chemical reduction (e.g. with Sn(II) chloride/HCI) of the nitro compound of formula ( II ) (q.v. Houben-Weyl, "Methoden d. org. Chemie" 11/1, 422). The nitro compound of formula ( II ) itself can be prepared by haloalkylating the suitably substituted nitrophenol. A further process for the preparation of the aniline of formula ( I ) comprises haloalkylating in corresponding manner acylated a suitably substituted aniline and then removing the acyl group, e.g. by acid hydrolysis, or effecting the haloalkylation with a salt of a suitably substituted aniline, e.g. the chlorohydrate. Subsequent one can introduce the substituents X₁ and X₂ by methods analogous to known ones, e.g. by reacting an aniline of the formula ( I ) with C₁-C₆chloroalkyl. The starting materials of formulae ( II ) are known and can be prepared by methods analogous to known ones.

Compounds of the formula ( I ) are described in many publications, mostly as intermediates or as starting material for the production of active ingredients for different uses: W09825466 describes 3-chloro-4-(1,2,2-trifluoro-2-trifluoromethoxyethoxy)-aniline; WO9819995 describes 2-fluoro-3,5-dichloro-4-(1 -methyl-2,2,3,3,3-pentafluoropropoxy)aniline; WO9819543 describes 3,5-dichloro-4-(1,1,2,3,3,3-hexafluoropropoxy)aniline and 3,5-dichloro-4-(1,1,2,3,3,3-hexafluoropropoxy)-N-ethyl-aniline; EP-179,022 describes 2,5-dichloro-4-(1,1,2,3,3,3-hexafluoropropoxy)aniline; WO98/19994 describes 3,5-dichloro-4-trans-(1,1,2,3,4,4,4-heptafluorobut-2-enoxy)aniline; WO9819542 describes 3,5-dichloro-4-(1,1,2,3,3,3-hexafluoropropoxy)aniline); further aniline derivatives of the formula ( I ) are described as starting material in EP-127,990.

From the above description, one skilled in the art can easily ascertain the essential characteristics of the present invention, and without departing from the spirit and scope thereof, can make various changes and modifications of the invention to adapt it to various utilities and conditions. Thus other embodiments are also within the claims.

Without further elaboration, it is believed that one skilled in the art can, based on the description herein, utilize the present invention to its fullest extent. The following specific example is, therefore, to be construed merely as illustrative, and does not limit the remainder of the disclosure in any way whatsoever. Publications mentioned herein are hereby incorporated by reference.

### Chemical Examples

**Table 1:**

| Typical representatives of the formula ( I ) | | | | | | | | |
|---|---|---|---|---|---|---|---|---|
| No. | R₁ | R₂ | R₃ | R₄ | X₁ | X₂ | Y | m.p. [°C] |
| 1 | H | H | Br | H | H | H | C₂F₅ | liquid |
| 2 | H | Cl | Cl | H | H | H | CF₂CHFCF₃ | liquid |
| 3 | H | Cl | F | H | H | H | CF₂CHFCF₃ | liquid |
| 4 | H | Br | F | H | H | H | CF₂CHFCF₃ | liquid |
| 5 | Cl | CF₃ | H | Cl | H | H | CF₂CHFCF₃ | liquid |
| 6 | F | H | F | H | H | H | CF₂CHFCF₃ | liquid |
| 7 | H | Cl | Cl | H | H | H | C₃F₇-n | liquid |
| 8 | F | Cl | H | Cl | H | H | CF₂CHFCF₃ | liquid |
| 9 | Cl | Cl | H | Cl | H | H | CF₂CHFCF₃ | liquid |
| 10 | F | F | F | F | H | H | CF₂CHFCF₃ | liquid |
| 11 | H | Cl | Cl | H | H | H | CF=CFCF₃ | liquid |
| 12 | H | Cl | Cl | H | H | H | CF₂CHFBr | liquid |
| 13 | H | Cl | Cl | H | H | H | CF₂CH₂F | 33-34 |
| 14 | H | Cl | Cl | H | H | H | CF₂CHF₂ | liquid |
| 15 | H | Cl | Cl | H | H | H | C₃F₇-n | liquid |
| 16 | H | Cl | Cl | H | H | H | CF₂CH₃ | 48-51 |
| 17 | Cl | Cl | Cl | Cl | H | H | CF₂CHCl₂ | 66-67 |
| 18 | CF₃ | H | H | H | H | H | CF₂CHFCF₃ | liquid |
| 19 | Cl | CF₃ | H | H | H | H | CF₂CHFCF₃ | liquid |
| 20 | CH₃ | H | CH₃ | H | H | H | CF₂CHFCF₃ | liquid |
| 21 | Cl | H | H | Cl | H | H | CF₂CHFCF₃ | liquid |
| 22 | CF₃ | H | Cl | H | H | H | CF₂CHFCF₃ | liquid |
| 23 | CF₃ | Cl | H | Cl | H | H | CF₂CHFCF₃ | liquid |
| 24 | Cl | CF₃ | H | Cl | H | H | CF₂CHFCF₃ | liquid |
| 25 | CF₃ | Cl | H | H | H | H | CF₂CHFCF₃ | liquid |
| 26 | Br | CF₃ | H | H | H | H | CF₂CHFCF₃ | liquid |
| 27 | H | H | H | H | H | H | CF₂CHFCF₃ | liquid |
| 28 | F | H | H | H | H | H | CF₂CHFCF₃ | liquid |
| 29 | Cl | Cl | H | Cl | H | H | CF₂CHFCF₃ | liquid |
| 30 | H | Cl | H | H | H | H | CF₂CHFCF₃ | liquid |
| 31 | Cl | Cl | Cl | Cl | H | H | C₂F₅ | |
| 32 | Cl | Br | Cl | Br | H | H | C₂F₅ | |
| 33 | H | Cl | Cl | Cl | CH₃ | CH₃ | CF₂CHFCF₃ | |
| 34 | H | Cl | F | Cl | CH₃ | CH₃ | CF₂CHFCF₃ | |
| 35 | H | Br | F | Cl | CH₃ | CH₃ | CF₂CHFCF₃ | |
| 36 | Cl | CF₃ | H | Cl | CH₃ | CH₃ | CF₂CHFCF₃ | |
| 37 | F | H | F | H | H | C₃H₇-n | CF₂CHFCF₃ | |
| 38 | F | Cl | H | Cl | H | C₃H₇-n | CF₂CHFCF₃ | |
| 39 | Cl | Cl | H | Cl | H | C₄H₉-n | CF₂CHFCF₃ | |
| 40 | F | F | F | F | H | C₄H₉-n | CF₂CHFCF₃ | |
| 41 | CF₃ | H | H | H | H | C₄H₉-s | CF₂CHFCF₃ | |
| 42 | Cl | CF₃ | H | H | H | H | CF₂CHFCF₃ | |
| 43 | CH₃ | H | CH₃ | H | H | H | CF₂CHFCF₃ | |
| 44 | Cl | H | H | Cl | H | H | CF₂CHFCF₃ | |
| 45 | CF₃ | H | Cl | H | H | H | CF₂CHFCF₃ | |
| 46 | CF₃ | Cl | H | Cl | H | H | CF₂CHFCF₃ | |
| 47 | Cl | CF₃ | H | Cl | H | H | CF₂CHFCF₃ | |
| 48 | CF₃ | Cl | H | H | H | H | CF₂CHFCF₃ | |
| 49 | Br | CF₃ | H | H | H | H | CF₂CHFCF₃ | |
| 50 | H | H | H | H | H | H | CF₂CHFCF₃ | |
| 51 | F | H | H | H | H | H | CF₂CHFCF₃ | |
| 52 | Cl | Cl | H | Cl | H | H | CF₂CHFCF₃ | |
| 53 | H | Cl | H | H | H | H | CF₂CHFCF₃ | |
| 54 | Cl | H | Cl | H | H | H | CF₂CHFCF₃ | |
| 55 | H | Cl | Cl | H | H | H | CH(CH₃)C₂F₅ | |
| 56 | H | Cl | F | H | H | H | CH(CH₃)C₂F₅ | |
| 57 | H | Br | F | H | H | H | CH(CH₃)C₂F₅ | |
| 58 | Cl | CF₃ | H | Cl | H | H | CH(CH₃)C₂F₅ | |
| 59 | F | H | F | H | H | H | CH(CH₃)C₂F₅ | |
| 60 | F | Cl | H | Cl | H | H | CH(CH₃)C₂F₅ | |
| 61 | Cl | Cl | H | Cl | H | H | CH(CH₃)C₂F₅ | |
| 62 | F | F | F | F | H | H | CH(CH₃)C₂F₅ | |
| 63 | CF₃ | H | H | H | H | H | CH(CH₃)C₂F₅ | |
| 64 | Cl | CF₃ | H | H | H | H | CH(CH₃)C₂F₅ | |
| 65 | CH₃ | H | CH₃ | H | H | H | CH(CH₃)C₂F₅ | |
| 66 | Cl | H | H | Cl | H | H | CH(CH₃)C₂F₅ | |
| 67 | CF₃ | H | Cl | H | H | H | CH(CH₃)C₂F₅ | |
| 68 | CF₃ | Cl | H | Cl | H | H | CH(CH₃)C₂F₅ | |
| 69 | Cl | CF₃ | H | Cl | H | H | CH(CH₃)C₂F₅ | |
| 70 | CF₃ | Cl | H | H | H | H | CH(CH₃)C₂F₅ | |
| 71 | Br | CF₃ | H | H | H | H | CH(CH₃)C₂F₅ | |
| 72 | H | H | H | H | H | H | CH(CH₃)C₂F₅ | |
| 73 | F | H | H | H | H | H | CH(CH₃)C₂F₅ | |
| 74 | Cl | Cl | H | Cl | H | H | CH(CH₃)C₂F₅ | |
| 75 | H | Cl | H | H | H | H | CH(CH₃)C₂F₅ | |
| 76 | Cl | H | Cl | H | H | H | CH(CH₃)C₂F₅ | |
| 77 | H | Cl | Cl | H | H | H | CH(CH₃)C₂F₄CF₃ | |
| 78 | H | Cl | F | H | H | H | CH(CH₃)C₂F₄CF₃ | |
| 79 | H | Br | F | H | H | H | CH(CH₃)C₂F₄CF₃ | |
| 80 | Cl | CF₃ | H | Cl | H | H | CH(CH₃)C₂F₄CF₃ | |
| 81 | F | H | F | H | H | H | CH(CH₃)C₂F₄CF₃ | |
| 82 | F | Cl | H | Cl | H | H | CH(CH₃)C₂F₄CF₃ | |
| 83 | Cl | Cl | H | Cl | H | H | CH(CH₃)C₂F₄CF₃ | |
| 84 | F | F | F | F | H | H | CH(CH₃)C₂F₄CF₃ | |
| 85 | CF₃ | H | H | H | H | H | CH(CH₃)C₂F₄CF₃ | |
| 86 | Cl | CF₃ | H | H | H | H | CH(CH₃)C₂F₄CF₃ | |
| 87 | CH₃ | H | CH₃ | H | H | H | CH(CH₃)C₂F₄CF₃ | |
| 88 | Cl | H | H | Cl | H | H | CH(CH₃)C₂F₄CF₃ | |
| 89 | CF₃ | H | Cl | H | H | H | CH(CH₃)C₂F₄CF₃ | |
| 90 | CF₃ | Cl | H | Cl | H | H | CH(CH₃)C₂F₄CF₃ | |
| 91 | Cl | CF₃ | H | Cl | H | H | CH(CH₃)C₂F₄CF₃ | |
| 92 | CF₃ | Cl | H | H | H | H | CH(CH₃)C₂F₄CF₃ | |
| 93 | Br | CF₃ | H | H | H | H | CH(CH₃)C₂F₄CF₃ | |
| 94 | H | H | H | H | H | H | CH(CH₃)C₂F₄CF₃ | |
| 95 | F | H | H | H | H | H | CH(CH₃)C₂F₄CF₃ | |
| 96 | Cl | Cl | H | Cl | H | H | CH(CH₃)C₂F₄CF₃ | |
| 97 | H | Cl | H | H | H | H | CH(CH₃)C₂F₄CF₃ | |
| 98 | Cl | H | Cl | H | H | H | CH(CH₃)C₂F₄CF₃ | |
| 99 | H | Cl | F | H | H | H | CF=CFCF₃ | |
| 100 | H | Br | F | H | H | H | CF=CFCF₃ | |
| 101 | Cl | CF₃ | H | Cl | H | H | CF=CFCF₃ | |
| 102 | F | H | F | H | H | H | CF=CFCF₃ | |
| 103 | F | Cl | H | Cl | H | H | CF=CFCF₃ | |
| 104 | Cl | Cl | H | Cl | H | H | CF=CFCF₃ | |
| 105 | F | F | F | F | H | H | CF=CFCF₃ | |
| 106 | CF₃ | H | H | H | H | H | CF=CFCF₃ | |
| 107 | Cl | CF₃ | H | H | H | H | CF=CFCF₃ | |
| 108 | CH₃ | H | CH₃ | H | H | H | CF=CFCF₃ | |
| 109 | Cl | H | H | Cl | H | H | CF=CFCF₃ | |
| 110 | CF₃ | H | Cl | H | H | H | CF=CFCF₃ | |
| 111 | CF₃ | Cl | H | Cl | H | H | CF=CFCF₃ | |
| 112 | Cl | CF₃ | H | Cl | H | H | CF=CFCF₃ | |
| 113 | CF₃ | Cl | H | H | H | H | CF=CFCF₃ | |
| 114 | Br | CF₃ | H | H | H | H | CF=CFCF₃ | |
| 115 | H | H | H | H | H | H | CF=CFCF₃ | |
| 116 | F | H | H | H | H | H | CF=CFCF₃ | |
| 117 | Cl | Cl | H | Cl | H | H | CF=CFCF₃ | |
| 118 | H | Cl | H | H | H | H | CF=CFCF₃ | |
| 119 | Cl | H | Cl | H | H | H | CF=CFCF₃ | |
| 120 | H | Cl | Cl | H | H | H | CF₂CF₂=CFCF₃ | |
| 121 | H | Cl | F | H | H | H | CF₂CF₂=CFCF₃ | |
| 122 | H | Br | F | H | H | H | CF₂CF₂=CFCF₃ | |
| 123 | Cl | CF₃ | H | Cl | H | H | CF₂CF₂=CFCF₃ | |
| 124 | F | H | F | H | H | H | CF₂CF₂=CFCF₃ | |
| 125 | F | Cl | H | Cl | H | H | CF₂CF₂=CFCF₃ | |
| 126 | Cl | Cl | H | Cl | H | H | CF₂CF₂=CFCF₃ | |
| 127 | F | F | F | F | H | H | CF₂CF₂=CFCF₃ | |
| 128 | CF₃ | H | H | H | H | H | CF₂CF₂=CFCF₃ | |
| 129 | Cl | CF₃ | H | H | H | H | CF₂CF₂=CFCF₃ | |
| 130 | CH₃ | H | CH₃ | H | H | H | CF₂CF₂=CFCF₃ | |
| 131 | Cl | H | H | Cl | H | H | CF₂CF₂=CFCF₃ | |
| 132 | CF₃ | H | Cl | H | H | H | CF₂CF₂=CFCF₃ | |
| 133 | CF₃ | Cl | H | Cl | H | H | CF₂CF₂=CFCF₃ | |
| 134 | Cl | CF₃ | H | Cl | H | H | CF₂CF₂=CFCF₃ | |
| 135 | CF₃ | Cl | H | H | H | H | CF₂CF₂=CFCF₃ | |
| 136 | Br | CF₃ | H | H | H | H | CF₂CF₂=CFCF₃ | |
| 137 | H | H | H | H | H | H | CF₂CF₂=CFCF₃ | |
| 138 | F | H | H | H | H | H | CF₂CF₂=CFCF₃ | |
| 139 | Cl | Cl | H | Cl | H | H | CF₂CF₂=CFCF₃ | |
| 140 | H | Cl | H | H | H | H | CF₂CF₂=CFCF₃ | |
| 141 | Cl | H | Cl | H | H | H | CF₂CF₂=CFCF₃ | |
| 142 | H | Cl | Cl | H | H | H | CF₂CFHOCF₃ | |
| 143 | H | Cl | F | H | H | H | CF₂CFHOCF₃ | |
| 144 | H | Br | F | H | H | H | CF₂CFHOCF₃ | |
| 145 | Cl | CF₃ | H | Cl | H | H | CF₂CFHOCF₃ | |
| 146 | F | H | F | H | H | H | CF₂CFHOCF₃ | |
| 147 | F | Cl | H | Cl | H | H | CF₂CFHOCF₃ | |
| 148 | Cl | Cl | H | Cl | H | H | CF₂CFHOCF₃ | |
| 149 | F | F | F | F | H | H | CF₂CFHOCF₃ | |
| 150 | CF₃ | H | H | H | H | H | CF₂CFHOCF₃ | |
| 151 | Cl | CF₃ | H | H | H | H | CF₂CFHOCF₃ | |
| 152 | CH₃ | H | CH₃ | H | H | H | CF₂CFHOCF₃ | |
| 153 | Cl | H | H | Cl | H | H | CF₂CFHOCF₃ | |
| 154 | CF₃ | H | Cl | H | H | H | CF₂CFHOCF₃ | |
| 155 | CF₃ | Cl | H | Cl | H | H | CF₂CFHOCF₃ | |
| 156 | Cl | CF₃ | H | Cl | H | H | CF₂CFHOCF₃ | |
| 157 | CF₃ | Cl | H | H | H | H | CF₂CFHOCF₃ | |
| 158 | Br | CF₃ | H | H | H | H | CF₂CFHOCF₃ | |
| 159 | H | H | H | H | H | H | CF₂CFHOCF₃ | |
| 160 | F | H | H | H | H | H | CF₂CFHOCF₃ | |
| 161 | Cl | Cl | H | Cl | H | H | CF₂CFHOCF₃ | |
| 162 | H | Cl | H | H | H | H | CF₂CFHOCF₃ | |
| 163 | Cl | H | Cl | H | H | H | CF₂CFHOCF₃ | |
| 164 | H | Cl | Cl | H | H | H | CF₃ | |
| 165 | H | Cl | F | H | H | H | CF₃ | |
| 166 | H | Br | F | H | H | H | CF₃ | |
| 167 | Cl | CF₃ | H | Cl | H | H | CF₃ | |
| 168 | F | H | F | H | H | H | CF₃ | |
| 169 | F | Cl | H | Cl | H | H | CF₃ | |
| 170 | Cl | Cl | H | Cl | H | H | CF₃ | |
| 171 | F | F | F | F | H | H | CF₃ | |
| 172 | CF₃ | H | H | H | H | H | CF₃ | |
| 173 | Cl | CF₃ | H | H | H | H | CF₃ | |
| 174 | CH₃ | H | CH₃ | H | H | H | CF₃ | |
| 175 | Cl | H | H | Cl | H | H | CF₃ | |
| 176 | CF₃ | H | Cl | H | H | H | CF₃ | |
| 177 | CF₃ | Cl | H | Cl | H | H | CF₃ | |
| 178 | Cl | CF₃ | H | Cl | H | H | CF₃ | |
| 179 | CF₃ | Cl | H | H | H | H | CF₃ | |
| 180 | Br | CF₃ | H | H | H | H | CF₃ | |
| 181 | H | H | H | H | H | H | CF₃ | |
| 182 | F | H | H | H | H | H | CF₃ | |
| 183 | Cl | Cl | H | Cl | H | H | CF₃ | |
| 184 | H | Cl | H | H | H | H | CF₃ | |
| 185 | Cl | H | Cl | H | H | H | CF₃ | |
| 186 | H | Cl | Cl | H | H | H | CF₂CHClF | |
| 187 | H | Cl | F | H | H | H | CF₂CHClF | |
| 188 | H | Br | F | H | H | H | CF₂CHClF | |
| 189 | Cl | CF₃ | H | Cl | H | H | CF₂CHClF | |
| 190 | F | H | F | H | H | H | CF₂CHClF | |
| 191 | F | Cl | H | Cl | H | H | CF₂CHClF | |
| 192 | Cl | Cl | H | Cl | H | H | CF₂CHClF | |
| 193 | F | F | F | F | H | H | CF₂CHClF | |
| 194 | CF₃ | H | H | H | H | H | CF₂CHClF | |
| 195 | Cl | CF₃ | H | H | H | H | CF₂CHClF | |
| 196 | CH₃ | H | CH₃ | H | H | H | CF₂CHClF | |
| 197 | Cl | H | H | Cl | H | H | CF₂CHClF | |
| 198 | CF₃ | H | Cl | H | H | H | CF₂CHClF | |
| 199 | CF₃ | Cl | H | Cl | H | H | CF₂CHClF | |
| 200 | Cl | CF₃ | H | Cl | H | H | CF₂CHClF | |
| 201 | CF₃ | Cl | H | H | H | H | CF₂CHClF | |
| 202 | Br | CF₃ | H | H | H | H | CF₂CHClF | |
| 203 | H | H | H | H | H | H | CF₂CHClF | |
| 204 | F | H | H | H | H | H | CF₂CHClF | |
| 205 | Cl | Cl | H | Cl | H | H | CF₂CHClF | |
| 206 | H | Cl | H | H | H | H | CF₂CHClF | |
| 207 | Cl | H | Cl | H | H | H | CF₂CHClF | |
| 208 | H | Cl | Cl | H | H | H | CF₂CHCHCl₂ | |
| 209 | H | Cl | F | H | H | H | CF₂CHCHCl₂ | |
| 210 | H | Br | F | H | H | H | CF₂CHCHCl₂ | |
| 211 | Cl | CF₃ | H | Cl | H | H | CF₂CHCHCl₂ | |
| 212 | F | H | F | H | H | H | CF₂CHCHCl₂ | |
| 213 | F | Cl | H | Cl | H | H | CF₂CHCHCl₂ | |
| 214 | Cl | Cl | H | Cl | H | H | CF₂CHCHCl₂ | |
| 215 | F | F | F | F | H | H | CF₂CHCHCl₂ | |
| 216 | CF₃ | H | H | H | H | H | CF₂CHCHCl₂ | |
| 217 | Cl | CF₃ | H | H | H | H | CF₂CHCHCl₂ | |
| 218 | CH₃ | H | CH₃ | H | H | H | CF₂CHCHCl₂ | |
| 219 | Cl | H | H | Cl | H | H | CF₂CHCHCl₂ | |
| 220 | CF₃ | H | Cl | H | H | H | CF₂CHCHCl₂ | |
| 221 | CF₃ | Cl | H | Cl | H | H | CF₂CHCHCl₂ | |
| 222 | Cl | CF₃ | H | Cl | H | H | CF₂CHCHCl₂ | |
| 223 | CF₃ | Cl | H | H | H | H | CF₂CHCHCl₂ | |
| 224 | Br | CF₃ | H | H | H | H | CF₂CHCHCl₂ | |
| 225 | H | H | H | H | H | H | CF₂CHCHCl₂ | |
| 226 | F | H | H | H | H | H | CF₂CHCHCl₂ | |
| 227 | Cl | Cl | H | Cl | H | H | CF₂CHCHCl₂ | |
| 228 | H | Cl | H | H | H | H | CF₂CHCHCl₂ | |
| 229 | Cl | H | Cl | H | H | H | CF₂CHCHCl₂ | |
| 230 | H | Cl | Cl | H | H | H | CF₂CHCFBr | |
| 231 | H | Cl | F | H | H | H | CF₂CHCFBr | |
| 232 | H | Br | F | H | H | H | CF₂CHCFBr | |
| 234 | Cl | CF₃ | H | Cl | H | H | CF₂CHCFBr | |
| 235 | F | H | F | H | H | H | CF₂CHCFBr | |
| 236 | F | Cl | H | Cl | H | H | CF₂CHCFBr | |
| 237 | Cl | Cl | H | Cl | H | H | CF₂CHCFBr | |
| 238 | F | F | F | F | H | H | CF₂CHCFBr | |
| 230 | CF₃ | H | H | H | H | H | CF₂CHCFBr | |
| 240 | Cl | CF₃ | H | H | H | H | CF₂CHCFBr | |
| 241 | CH₃ | H | CH₃ | H | H | H | CF₂CHCFBr | |
| 242 | Cl | H | H | Cl | H | H | CF₂CHCFBr | |
| 243 | CF₃ | H | Cl | H | H | H | CF₂CHCFBr | |
| 244 | CF₃ | Cl | H | Cl | H | H | CF₂CHCFBr | |
| 245 | Cl | CF₃ | H | Cl | H | H | CF₂CHCFBr | |
| 246 | CF₃ | Cl | H | H | H | H | CF₂CHCFBr | |
| 247 | Br | CF₃ | H | H | H | H | CF₂CHCFBr | |
| 248 | H | H | H | H | H | H | CF₂CHCFBr | |
| 249 | F | H | H | H | H | H | CF₂CHCFBr | |
| 250 | Cl | Cl | H | Cl | H | H | CF₂CHCFBr | |
| 251 | H | Cl | H | H | H | H | CF₂CHCFBr | |
| 252 | Cl | H | Cl | H | H | H | CF₂CHCFBr | |
| 253 | H | Cl | Cl | H | H | H | CF₂CHFCF₃ | |
| 254 | H | Cl | F | H | H | H | CF₂CHFCF₃ | |
| 255 | H | Br | F | H | H | H | CF₂CHFCF₃ | |
| 256 | Cl | CF₃ | H | Cl | H | H | CF₂CHFCF₃ | |
| 257 | F | H | F | H | H | H | CF₂OCF₃ | |
| 258 | F | Cl | H | Cl | H | H | CF₂OCF₃ | |
| 259 | Cl | Cl | H | Cl | H | H | CF₂OCF₃ | |
| 260 | F | F | F | F | H | H | CF₂OCF₃ | |
| 261 | CF₃ | H | H | H | H | H | CF₂OCF₃ | |
| 262 | Cl | CF₃ | H | H | H | H | CF₂OCF₃ | |
| 263 | CH₃ | H | CH₃ | H | H | H | CF₂OCF₃ | |
| 264 | Cl | H | H | Cl | H | H | CF₂OCF₃ | |
| 265 | CF₃ | H | Cl | H | H | H | CF₂OCF₃ | |
| 266 | CF₃ | Cl | H | Cl | H | H | CF₂OCF₃ | |
| 267 | Cl | CF₃ | H | Cl | H | H | CF₂OCF₃ | |
| 268 | CF₃ | Cl | H | H | H | H | CF₂OCF₃ | |
| 269 | Br | CF₃ | H | H | H | H | CF₂OCF₃ | |
| 270 | H | H | H | H | H | H | CF₂OCF₃ | |
| 271 | F | H | H | H | H | H | CF₂OCF₃ | |
| 272 | Cl | Cl | H | Cl | H | H | CF₂OCF₃ | |
| 273 | H | Cl | H | H | H | H | CF₂OCF₃ | |
| 274 | Cl | H | Cl | H | H | H | CF₂OCF₃ | |
| 275 | H | H | Br | H | CH₃ | H | C₂F₅ | |
| 276 | H | Cl | Cl | H | CH₃ | H | C₂F₅ | |
| 277 | H | Cl | F | H | CH₃ | H | C₂F₅ | |
| 278 | H | Br | F | H | CH₃ | H | C₂F₅ | |
| 279 | Cl | CF₃ | H | Cl | CH₃ | H | C₂F₅ | |
| 280 | F | H | F | H | CH₃ | H | C₂F₅ | |
| 281 | H | Cl | Cl | H | CH₃ | H | C₃F₇-n | |
| 282 | F | Cl | H | Cl | CH₃ | H | C₂F₅ | |
| 283 | Cl | Cl | H | Cl | CH₃ | H | C₂F₅ | |
| 284 | F | F | F | F | CH₃ | H | C₂F₅ | |
| 285 | H | Cl | Cl | H | CH₃ | H | CF=CFCF₃ | |
| 286 | H | Cl | Cl | H | CH₃ | H | CF₂CHFBr | |
| 287 | H | Cl | Cl | H | CH₃ | H | CF₂CH₂F | |
| 289 | H | Cl | Cl | H | CH₃ | H | CF₂CHF₂ | |
| 290 | H | Cl | Cl | H | CH₃ | H | C₃F₇-n | |
| 291 | H | Cl | Cl | H | CH₃ | H | CF₂CH₃ | |
| 292 | Cl | Cl | Cl | Cl | CH₃ | H | CF₂CHCl₂ | |
| 293 | CF₃ | H | H | H | CH₃ | H | C₂F₅ | |
| 294 | Cl | CF₃ | H | H | CH₃ | H | C₂F₅ | |
| 295 | CH₃ | H | CH₃ | H | CH₃ | H | C₂F₅ | |
| 296 | Cl | H | H | Cl | CH₃ | H | C₂F₅ | |
| 297 | CF₃ | H | Cl | H | CH₃ | H | C₂F₅ | |
| 298 | CF₃ | Cl | H | Cl | CH3 | H | C₂F₅ | |
| 299 | Cl | CF₃ | H | Cl | CH₃ | H | C₂F₅ | |
| 300 | CF₃ | Cl | H | H | CH₃ | H | C₂F₅ | |
| 301 | Br | CF₃ | H | H | CH₃ | H | C₂F₅ | |
| 302 | H | H | H | H | CH₃ | H | C₂F₅ | |
| 303 | F | H | H | H | CH₃ | H | C₂F₅ | |
| 304 | Cl | Cl | H | Cl | CH₃ | H | C₂F₅ | |
| 305 | H | Cl | H | H | CH₃ | H | C₂F₅ | |
| 306 | Cl | Cl | Cl | Cl | CH₃ | H | C₂F₅ | |
| 307 | Cl | Br | CL | Br | CH₃ | H | C₂F₅ | |
| 308 | H | Cl | Cl | H | CH₃ | H | CF₂CHFCF₃ | |
| 309 | H | Cl | F | H | CH₃ | H | CF₂CHFCF₃ | |
| 310 | H | Br | F | H | CH₃ | H | CF₂CHFCF₃ | |
| 311 | Cl | CF₃ | H | Cl | CH₃ | H | CF₂CHFCF₃ | |
| 312 | F | H | F | H | CH₃ | H | CF₂CHFCF₃ | |
| 313 | F | Cl | H | Cl | CH₃ | H | CF₂CHFCF₃ | |
| 314 | Cl | Cl | H | Cl | CH₃ | H | CF₂CHFCF₃ | |
| 315 | F | F | F | F | CH₃ | H | CF₂CHFCF₃ | |
| 316 | CF₃ | H | H | H | CH₃ | H | CF₂CHFCF₃ | |
| 317 | Cl | CF₃ | H | H | CH₃ | H | CF₂CHFCF₃ | |
| 318 | CH₃ | H | CH₃ | H | CH₃ | H | CF₂CHFCF₃ | |
| 319 | Cl | H | H | Cl | CH₃ | H | CF₂CHFCF₃ | |
| 320 | CF₃ | H | Cl | H | CH₃ | H | CF₂CHFCF₃ | |
| 321 | CF₃ | Cl | H | Cl | CH₃ | H | CF₂CHFCF₃ | |
| 322 | Cl | CF₃ | H | Cl | CH₃ | H | CF₂CHFCF₃ | |
| 323 | CF₃ | Cl | H | H | CH₃ | H | CF₂CHFCF₃ | |
| 324 | Br | CF₃ | H | H | CH₃ | H | CF₂CHFCF₃ | |
| 325 | H | H | H | H | CH₃ | H | CF₂CHFCF₃ | |
| 326 | F | H | H | H | CH₃ | H | CF₂CHFCF₃ | |
| 327 | Cl | Cl | H | Cl | CH₃ | H | CF₂CHFCF₃ | |
| 328 | H | Cl | H | H | CH₃ | H | CF₂CHFCF₃ | |
| 329 | Cl | H | Cl | H | CH₃ | H | CF₂CHFCF₃ | |
| 330 | H | Cl | Cl | H | CH₃ | H | CH(CH₃)C₂F₅ | |
| 331 | H | H | Br | H | CH₃ | C₂H₅ | C₂F₅ | |
| 332 | H | Cl | Cl | H | CH₃ | CH₃ | C₂F₅ | |
| 333 | H | Cl | F | H | CH₃ | CH₃ | C₂F₅ | |
| 334 | H | Br | F | H | CH₃ | CH₃ | C₂F₅ | |
| 335 | Cl | CF₃ | H | Cl | CH₃ | CH₃ | C₂F₅ | |
| 336 | F | H | F | H | CH₃ | C₂H₅ | C₂F₅ | |
| 337 | H | Cl | Cl | H | CH₃ | CH₃ | C₃F₇-n | |
| 338 | F | Cl | H | Cl | CH₃ | CH₃ | C₂F₅ | |
| 339 | Cl | Cl | H | Cl | CH₃ | C₂H₅ | C₂F₅ | |
| 340 | F | F | F | F | CH₃ | CH₃ | C₂F₅ | |
| 341 | H | Cl | Cl | H | CH₃ | CH₃ | CF=CFCF₃ | |
| 342 | H | Cl | Cl | H | CH₃ | CH₃ | CF₂CHFBr | |
| 343 | H | Cl | Cl | H | CH₃ | C₂H₅ | CF₂CH₂F | |
| 344 | H | Cl | Cl | H | CH₃ | CH₃ | CF₂CHF₂ | |
| 345 | H | Cl | Cl | H | CH₃ | C₂H₅ | C₃F₇-n | |
| 346 | H | Cl | Cl | H | CH₃ | CH₃ | CF₂CH₃ | |
| 347 | Cl | Cl | Cl | Cl | CH₃ | CH₃ | CF₂CHCl₂ | |
| 348 | CF₃ | H | H | H | CH₃ | CH₃ | C₂F₅ | |
| 349 | Cl | CF₃ | H | H | CH₃ | C₂H₅ | C₂F₅ | |
| 350 | CH₃ | H | CH₃ | H | CH₃ | C₂H₅ | C₂F₅ | |
| 351 | Cl | H | H | Cl | CH₃ | CH₃ | C₂F₅ | |
| 352 | CF₃ | H | Cl | H | CH₃ | C₂H₅ | C₂F₅ | |
| 353 | CF₃ | Cl | H | Cl | CH₃ | CH₃ | C₂F₅ | |
| 354 | Cl | CF₃ | H | Cl | CH₃ | CH₃ | C₂F₅ | |
| 355 | CF₃ | Cl | H | H | CH₃ | CH₃ | C₂F₅ | |
| 356 | Br | CF₃ | H | H | CH₃ | CH₃ | C₂F₅ | |
| 357 | H | H | H | H | CH₃ | C₂H₅ | C₂F₅ | |
| 358 | F | H | H | H | CH₃ | CH₃ | C₂F₅ | |
| 359 | Cl | Cl | H | Cl | CH₃ | C₂H₅ | C₂F₅ | |
| 360 | H | Cl | H | H | CH₃ | CH₃ | C₂F₅ | |
| 361 | Cl | Cl | Cl | Cl | CH₃ | CH₃ | C₂F₅ | |
| 362 | Cl | Br | CL | Br | CH₃ | CH₃ | C₂F₅ | |
| 363 | H | Cl | Cl | H | CH₃ | CH₃ | CF₂CHFCF₃ | |
| 364 | H | Cl | F | H | CH₃ | C₂H₅ | CF₂CHFCF₃ | |
| 365 | H | Br | F | H | CH₃ | CH₃ | CF₂CHFCF₃ | |
| 366 | Cl | CF₃ | H | Cl | CH₃ | C₂H₅ | CF₂CHFCF₃ | |
| 367 | F | H | F | H | CH₃ | CH₃ | CF₂CHFCF₃ | |
| 368 | F | Cl | H | Cl | CH₃ | CH₃ | CF₂CHFCF₃ | |
| 369 | Cl | Cl | H | Cl | CH₃ | CH₃ | CF₂CHFCF₃ | |
| 370 | F | F | F | F | CH₃ | CH₃ | CF₂CHFCF₃ | |
| 371 | CF₃ | H | H | H | CH₃ | CH₃ | CF₂CHFCF₃ | |
| 372 | Cl | CF₃ | H | H | CH₃ | CH₃ | CF₂CHFCF₃ | |
| 373 | CH₃ | H | CH₃ | H | CH₃ | CH₃ | CF₂CHFCF₃ | |
| 374 | Cl | H | H | Cl | CH₃ | CH₃ | CF₂CHFCF₃ | |
| 375 | CF₃ | H | Cl | H | CH₃ | CH₃ | CF₂CHFCF₃ | |
| 376 | CF₃ | Cl | H | Cl | CH₃ | CH₃ | CF₂CHFCF₃ | |
| 377 | Cl | CF₃ | H | Cl | CH₃ | CH₃ | CF₂CHFCF₃ | |
| 378 | CF₃ | Cl | H | H | CH₃ | CH₃ | CF₂CHFCF₃ | |
| 379 | Br | CF₃ | H | H | CH₃ | CH₃ | CF₂CHFCF₃ | |
| 380 | H | H | H | H | CH₃ | CH₃ | CF₂CHFCF₃ | |
| 381 | F | H | H | H | CH₃ | CH₃ | CF₂CHFCF₃ | |
| 382 | Cl | Cl | H | Cl | CH₃ | CH₃ | CF₂CHFCF₃ | |
| 383 | H | Cl | H | H | CH₃ | CH₃ | CF₂CHFCF₃ | |
| 384 | Cl | H | Cl | H | CH₃ | CH₃ | CF₂CHFCF₃ | |
| 385 | H | Cl | Cl | H | CH₃ | CH₃ | CH(CH₃)C₂F₅ | |

In the following formulation examples of use in men, domestic animals, livestock, and pets, the term "active ingredient" is understood to mean one or more active ingredients of formula ( I ) or a salt thereof, and preferably 3,5-dichloro-4-(1,1,2,3,3,3-hexafluoropropoxy)aniline.
Tablets: containing one of the active ingredients of formula ( I ) can be prepared as follows:

| Composition (for 1000 tablets) | |
|---|---|
| Active ingredient of formula ( I ) | 25 g |
| Lactose | 100.7 g |
| Wheat starch | 6.25 g |
| Polyethylene glycol 6000 | 5.0 g |
| Talc | 5.0 g |
| Magnesium stearate | 1.8 g |
| Deionized water | q.s. |

Preparation: All solid ingredients are first passed through a sieve with a mesh size of 0.6 mm. The active ingredient, the lactose, the talc, and half the starch are then mixed. The other half of the starch is suspended in 40 ml water, and this suspension is added to a boiling solution of the polyethylene glycol in 100 ml water. The resulting starch paste is added to the mixture, and this is then granulated, water being added where appropriate. The granulate is dried overnight at 35°, passed through a sieve with a mesh size of 1.2 mm, mixed with the magnesium stearate, and compressed to form tablets concave on both sides and with a diameter of 6 mm.

Tablets: each containing a total of 0.0183 g active ingredient are prepared as follows:

| Composition (for 10,000 tablets) | |
|---|---|
| Active ingredient of formula (I) | 183.00 g |
| Lactose | 290.80 g |
| Potato starch | 274.70 g |
| Stearic acid | 10.00 g |
| Talc | 217.00 g |
| Magnesium stearate | 2.50 g |
| Colloidal silica | 32.00 g |
| Ethanol | q.s. |

A mixture of the active ingredient, the lactose and 274.70 g potato starch is wetted with an ethanolic solution of stearic acid and granulated through a sieve. After drying, the remaining potato starch, the talc, the magnesium stearate, and the colloidal silica are added and the mixture compressed to form tablets of 0.1 g each in weight, which - if so desired - can be scored to allow for a finer adjustment of the dose.

Capsules: each containing a total of 0.022 g active ingredient can be prepared as follows:

| Composition (for 1000 capsules) | |
|---|---|
| Active ingredient of formula (I) | 22.00 g |
| Lactose | 249.80 g |
| Gelatin | 2.00 g |
| Corn starch | 10.00 g |
| Talc | 15.00 g |
| Water | q.s. |

The active ingredient is mixed with the lactose, the mixture wetted evenly with an aqueous solution of the gelatin and granulated through a sieve with a mesh size of 1.2-1.5 mm. The granulate is mixed with the dried corn starch and the talc, and portions of 300 mg are filled into hard gelatin capsules (size 1).

### Premix (feed additive)

- 0.16: parts by weight of active ingredient
- 4.84: parts by weight of secondary calcium phosphate, alumina, aerosil, carbonate or calcium carbonate are mixed until homogeneous with
- 95: parts by weight of an animal feed or
- 0.41: parts by weight of active ingredient
- 5.00: parts by weight of aerosil / calcium carbonate (1:1) are mixed until homogeneous with
- 94.59: parts by weight of a commercially available feed.

| Boli: | | |
|---|---|---|
| I | Active ingredient | 33.00 % |
| | Methylcellulose | 0.80 % |
| | Silicic acid, highly dispersed | 0-80 % |
| | Corn starch | 8.40 % |
| II | Lactose, cryst. | 22.50 % |
| | Corn starch | 17.00 % |
| | Microcrist. cellulose | 16.50 % |
| | Magnesium stearate | 1.00 % |

The methylcellulose is first stirred into water. After the material has swollen, the silicic acid is stirred in and the mixture homogeneously suspended. The active ingredient and the corn starch are mixed. The aqueous suspension is worked into this mixture and kneaded to a dough. The resulting mass is granulated through a 12 M sieve and dried. In a further step, all 4 adjuvants are thoroughly mixed. Finally, the premixtures resulting from the first two partial steps are mixed and compressed to form boli.

### Injectables:

### A. Oily vehicle (slow release)

| | |
|---|---|
| Active ingredient | 0.1-1.0 g |
| Groundnut oil | ad 100 ml |
| or | |
| Active ingredient | 0.1-1.0 g |
| Sesame oil | ad 100 ml |

Preparation: The active ingredient is dissolved in part of the oil with stirring and where appropriate gentle heating, then made up to the desired volume and sterile-filtered through a suitable membrane filter with a pore size of 0.22 µm.

The following examples of preparation and application serve to explain the invention without limiting it to the individual aspects of these examples.

Solutions (For Dilution With Drinking Water):
15% active ingredient in 2,2-dimethyl-4-hydroxy methyl1-1,3-dioxolane
10% active ingredient in diethylene glycol monethyl ether
10% active ingredient in polyethylene glycol (mol. wt. 300)
5% active ingredient in glycerol

Soluble Powder:
25 parts of active ingredient
1 part of sodium lauryl sulfate
3 parts of colloidal silica
71 parts of urea

The constituents are mixed and the mixture is finely ground in a suitable mill. Other biocidal active ingredients or agents which are inert towards the active ingredients and acceptable to men or animals to be treated, or mineral salts or vitamins can be admixed to the compositions described.

### Biological Examples Methods and Materials

Pathogens: the fungi tested are a test panel designed to represent the major groups of fungal pathogens. Table 2 indicates some brief details. The dermatophytes are the major causes of skin and nail infections, Candida and Cryptococcus are important systemic pathogens of the yeast type, especially in immunocompromised patients. Candida is also important in mucosal and genital infections (very common). Aspergillus represents the filamentous pathogens, and is one of the most serious life-threatening infections in neutropenic patients (eg cancer therapy, BMT). Saccharomyces is included because this is a useful biological tool.

Tests are performed with a set of reference strains including 3 yeasts and 3 filamentous species, as listed in Table 2. Compound No. 2 of table 1 is used as a typical representative of the compounds of formula ( I ). The purity of the compound (analyzed in compliance with GLP standards) 98.5%. Terbinafine HCI is used as the reference standard for the antifungal tests.

**Table 2:**

| Fungal strains used in this study | | | |
|---|---|---|---|
| Fungus | Strain No. | Origin | |
| *Trichophyton mentagrophytes* | NFI 0158 | NFI collection | Pathogen (dermatophyte) of skin, hair, nails in humans and animals |
| *Microsporum canis* | NFI 0150 | NFI collection | Pathogen (dermatophyte) of skin, hair, in humans and animals. Major cause of tinea capitic (ringworm) in some areas |
| *Aspergillus fumigatus* | NFI 0159 | NFI collection | Filamentous pathogen, major cause of serious life-threatening infections in immunocompromised hosts, also allergic bronchial disease. |
| *Candida albicans* | NFI 0124 | NFI collection | Yeast, common human pathogen causing skin and mucosal infections, eg. Candida vaginitis, mucosal candidiasis in HIV+ patients, as well as life-threatening systemic infections. |
| *Cryptococcus neoformans* | NFI 3102 | ATCC 36556 | Yeast, agent of serious systemic infections, esp. meningitis, in (usually) immunocompromised patients. Occasional skin infections. |
| *Saccharomyces cerevisiae* | NFI 3201 | ATCC 9763 | Bakers yeast, (normally) non-pathogenic. Standard model organism for molecular studies. |

### Antifungal Testing

MICs (Minimum Inhibitory Concentrations) are determined in broth macrodilution assays according to the NCCLS M27-A protocol (1) with slight modifications as detailed in a previous report (2). Inocula for assays are prepared from stocks frozen at -80°C by dilution in growth medium to give a final viable cell count of 2.5x10³ CFU (colony-forming units)/ml. Each assay is performed with a duplicate series of drug dilutions. In brief, the assays are done in RPMI 1640 (Roswell Park Memorial Institute) medium buffered to pH 7.0 with MOPS (3-[N-morpholino]propanesulfonic acid) buffer, incubated at 35°C for 48 hours. *Cryptococcus* and *Aspergillus* are tested in the same assay, except that incubations are for 72 hours. The dermatophytes *T. mentagrophytes* and *M. canis* are incubated at 30°C for 7 days. The MIC (80%) is defined as the lowest concentration of drug causing 80% inhibition of fungal growth in comparison with untreated controls. The MIC (100%) is defined as the lowest concentration of drug causing complete inhibition of fungal growth (no visible growth). For determination of the Minimum Fungicidal Concentration (MFC), samples (0.1 ml) are removed from the assay tubes at the end of the incubation. In the case of filamentous fungi, cell viability is then checked by streaking onto Sabouraud 2% dextrose agar plates which are incubated at 30°C for 7 days. In the case of yeasts, the samples are inoculated into 5 ml of RPMI 1640 medium (as used for MICs) and incubated at 35°C for 48 hours (72 hours for *Cr. neoformans).* The MFC is defined as the lowest drug concentration at which no visible fungal growth developed.

### Results

**Table 3.**

| Typical representative of the formula ( I ) and terbinafine as an internal standard | | | | | | | | | |
|---|---|---|---|---|---|---|---|---|---|
| **Compound** | ***T. mentagrophytes*** | | | ***M. canis*** | | | ***A. fumigatus*** | | |
| | MIC 80% | MIC 100% | MFC | MIC 80% | MIC 100% | MFC | MIC 80% | MIC 100% | MFC |
| No. 2 (table 1) | 4 | 8 | 16 | 4 | 8 | 16 | 8 | 16 | 16 |
| Terbinafine | - | 0.008 | 0.008 | - | 0.016 | 0.016 | - | 2 | 4 |

| **Compound** | ***C. albicans*** | | | ***Cr. neoformans*** | | | ***S. cerevisiae*** | | |
|---|---|---|---|---|---|---|---|---|---|
| | MIC 80% | MIC 100% | MFC | MIC 80% | MIC 100% | MFC | MIC 80% | MIC 100% | MFC |
| No.2 (table 1) | 16 | 32 | 32 | 0.5 | >128 | >128 | 16 | 32 | 32 |
| Terbinafine | 1 | >128 | >128 | 0.125 | >128 | >128 | >128 | >128 | >128 |

### Discussion

As shown in Table 3, the reference drug terbinafine displays the expected activity in these strains in accordance with previous data. Compound No. 2 of Table 1 showed broad spectrum antifungal activity against both yeasts and filamentous fungi, with a primary fungicidal action in most cases. Minimum inhibitory concentrations ranged between 0.5 to 32 µg/ml. With the exception of *Cr. neoformans,* the compound causes 100% inhibition of growth and displays a primary fungicidal action.

### References

(1) National Committee for Clinical Laboratory Standards. Reference method for broth dilution antifungal susceptibility testing of yeasts; approved standard. NCCLS Document M27-A. NCCLS, Wayne, Pa, U.S.A. 1997;
(2). Ryder NS, Wagner S, Leitner I. In vitro activities of terbinafine against cutaneous isolates of *Candida albicans* and other pathogenic yeasts. Antimicrob.Agents Chemother. 1998;42:1057-61.

## Claims

1. Antimycotic compositions comprising as active ingredient a compound of the formula ( I ) 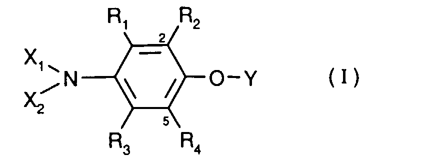 wherein Y is a completely or partially halogenated C₁-C₆alkyl or a completely or partially halogenated and with an oxygen atom interrupted C₁-C₆alkyl or a completely or partially halogenated C₂-C₆alkenyl; R₁, R₂, R₃ and R₄ are independently of each other hydrogen, halogen, C₁-C₆alkyl, C₁-C₆haloalkyl, C₁-C₆haloalkoxy, X₁ is hydrogen or C₁-C₆alkyl, X₂ is hydrogen or C₁-C₆alkyl.

2. Antimycotic compositions according to claim 1 comprising as active ingredient a compound of the formula ( I ) wherein Y is CF₃, C₂F₅, C₃F₇, C₄F₉, C₅F₁₁, C₆F₁₃, CF(CF₃)₂, CF(C₂F₅)(CF₃), CF(C₂F₅)(C₂F₅), CF₂OCF₃, CF₂OCF(C₂F₅)₂, CF₂CHFCF₃, CH(CF₃)CF₂CF₃, CH(CF₃)CF₂C₂F₅, CH(CH₃)CF₂CF₃, CH(CH₃)CF₂C₂F₅, CF=CFCF₃, CF₂CF=CFCF₃, CF₂(CF₃)CF₂=CFCF₃, OCF₂(CF₃)-O-CF₂=CFCF₃, CF₂CFHOCF₃, CF₂CCl₃, CF₂CHCl₂, CF₂CHF₂, CF₂CFCl₂, CF₂CHBr₂, CF₂CHClF, CH₂CHBrCH₂Br, CF₂CHBrF or CClFCHClF, X₁ and X₂ are independently of each other H, CH₃ or C₂H₅; and R₁, R₂, R₃ and R₄ are as defined as in claim 1.

3. Antimycotic compositions according to claim 1 comprising as active ingredient a compound of the formula ( I ) wherein Y is CF₂CHFCF₃, CH(CF₃)CF₂CF₃, CH(CF₃)CF₂C₂F₅, CH(CH₃)CF₂CF₃, CH(CH₃)CF₂C₂F₅, CF=CFCF₃, CF₂CF=CFCF₃, CF₂CHF₂, or CF₂CFHOCF₃; X₁ and X₂ are independently of each other H, CH₃ and C₂H₅; R₁ is H, F, Cl, Br, CH₃ or CF₃; R₂ is H, Cl, Br or CF₃, R₃ is H, F, Cl, Br or CH₃, and R₄ is H, Cl or Br.

4. Antimycotic compositions according to any one of claims 1 to 3 comprising as active ingredient a compound of the formula ( I ) wherein X₁ and X₂ are H.

5. Antimycotic compositions according to claim 1 comprising as active ingredient a compound of the formula ( I ) selected of the group consisting of 3-chloro-4-(1,2,2-trifluoro-2-trifluoromethoxyethoxy)-aniline; 2-fluoro-3,5-dichloro-4-(1-methyl-2,2,3,3,3-pentafluoropropoxy)aniline; 3,5-dichloro-4-(1,1,2,3,3,3-hexafluoropropoxy)aniline; 3,5-dichloro-4-(1,1,2,3,3,3-hexafluoropropoxy)-N-ethyl-aniline; 3,5-dichloro-4-trans-(1,1,2,3,4,4,4-heptafluorobut-2-enoxy)aniline; 3,5-dichloro-4-(1,1,2,3,3,3-hexafluoropropoxy)aniline); and 2,5-dichloro-4-(1,1,2,3,3,3-hexafluoropropoxy)aniline.

6. Use of a compound of the formula ( I ) as defined in any one of claims 1 to 5 for the treatment of mycotic infections in humans or animals.

7. Use of an antimycotic composition as defined in any one of claims 1 to 5 for the prevention or treatment of mycotic infections in humans or animals.

8. A method for the control of pathogenic fungi and yeasts in and on humans, domestic animals, livestock and pets, comprising a composition which contains at least one compound of formula ( I ) as defined in any one of claims 1 to 5, or a veterinarily acceptable salt thereof, and is administered to the host animal orally, parenterally or by implant at an effective dose.

9. A method according to claim 1 characterized in that the controlled disease is ringworm.

10. A compound of the formula ( I ) as defined in any one of claims 1 to 5 for the preparation of an antimycotic drug.

11. A compound of the formula ( I ) as defined in any one of claims 1 to 5 for the use in a method for the treatment of mycotic infections in humans or animals.
